# EUROPÄISCHE PATENTANMELDUNG

(11) **EP 0 995 428 A2**
(43) Veröffentlichungstag der Anmeldung: **26.04.2000**
(21) Anmeldenummer: 99116724.8
(22) Anmeldetag: 26.08.1999
(51) Int. Cl.: A61K 7/48

(54) **Retinoide enthaltende Hautpflegemittel**

(30) Priorität: 29.08.1998 DE 19839402
(71) Anmelder: Beiersdorf Aktiengesellschaft, 20245 Hamburg (DE)
(72) Erfinder: Sattler, Henning, Dr., 25836 Garding (DE); Zelle, Dagmar, 21640 Bliedersdorf (DE); Filbry, Alexander, Dr., 22459 Hamburg (DE)

(57) **Zusammenfassung**

Gegenstand der Erfindung sind O/W Emulsion mit einem Gehalt an Retinoiden, dadurch gekennzeichnet, daß sie einen Emulgator oder mehrere Emulgatoren aus der Gruppe, bestehend aus
a) einem Emulgator auf Basis von Glyceryl Stearat;
b) einem Emulgator auf Basis von Glyceryl Stearat und PEG-30 Stearat,
c) einem Emulgator auf Basis von Glyceryl Stearat und Ceteth-20,
d) einem Emulgator auf Basis von Cetearyl Alcohol und PEG-40 Castor Oil und Sodium Cetearyl Sulfat
e) einem Emulgator auf Basis von Sorbitan Stearat
enthält.

## Beschreibung

Gegenstand der Erfindung ist die Stabilisierung von Vitamin A und dessen Derivaten durch spezielle O/W-Emulgatoren in topisch anzuwendenden O/W-Emulsionen.

Die Erfindung betrifft physikalisch und chemisch stabile Retinoid-haltige Hautpflegemittel. Diese dienen dazu den Hautzustand in bekannter Weise zu verbessern. Während Vitamin A Säure-haltige Zubereitungen als relativ stabil gehen, sind Retinol (Vitamin A- bzw. Vitamin A₁-Alkohol) und Vitamin A-Ester wesentlich schwerer zu stabilisieren.
Vitamin A und dessen Derivate enthaltende Zubereitungen sollen den Hautzustand insbesondere älterer Haut verbessern und der Rauhigkeit, der Faltenbildung und den durch Sonnenlicht verursachten Schäden entgegenwirken. Viele derartige im Handel befindlichen Präparate weisen jedoch nicht immer die für eine gute Wirksamkeit erwünschte Stabilität des Vitamin A und dessen Estern, wie z. B. Vitamin A-Palmitat und -Acetat auf. Bei diesen Handelspräparaten handelt es sich überwiegend um O/W-Emulsionen.

Zur Stabilisierung von Retinoiden werden Antioxidantien und Chelatbildner eingesetzt. So beschreibt das US-Patent 3,906,108 die Verwendung von BHT oder DL-α-Tocopherol und EDTA für Vitamin A Säure in einer O/W-Emulsion.
Im US-Patent 4,247,547 für ein Gel mit Vitamin A Säure werden als Antioxidantien BHT, BHA, Ascorbinsäure, Propylgallat und DL-α-Tocopherol genannt.
Im US-Patent 4,446,805 wird eine O/W-Emulsion beschrieben, die unter anderem Vitamin A und als Stabilisatoren DL-α-Tocopherol und Zitronensäure enthält.
US-Patent 4,720,353, EP 0 343 444 (A2) und EP 0 330 496 (A2) haben die Verwendung der genannten Stabilisatoren für Vitamin A Ester in W/O-Emulsionen zum Inhalt.

Im WO 93/00085 wird die Stabilisierung einer W/O-Emulsion mit Vitamin A-Alkohol, Vitamin A-Aldehyd, Vitamin A-Acetat und Vitamin A-Palmitat beschrieben. Beansprucht wird die Verwendung eines Chelatbildners in Kombination mit jeweils einem öllöslichen und einem wasserlöslichen Antioxidans. Die W/O-Emulsionen werden als stabil bezeichnet, wenn sie nach 13 Wochen Lagerung bei 40 °C noch 60% des Retinoid-Ausgangswertes bzw. bei RT noch 80% enthalten.
Das russische Patent RU 203 66 40 beansprucht eine Salbenzusammensetzung, also eine W/O-Emulsion, mit Vitamin A-Palmitat und einem Antioxidans von der angegeben wird, daß das Vitamin A-Palmitat stabil sei.

Die genannten Lösungswege sind jedoch nicht immer voll befriedigend.

Wie schon erwähnt, sind Vitamin A und dessen Derivate vor allem in O/W-Emulsionen schwierig zu stabilisieren. Dies dürfte darin begründet sein, daß der Sauerstoff der Luft durch die äußere Wasserphase der Emulsion leicht diffundieren kann und so eine Zersetzung des Vitamin A oder -Derivates bewirkt.

Aufgabe der Erfindung war es, die vorstehenden Nachteile zu vermeiden.

Die trotz Anwesenheit von Antioxidantien und Chelatbildnern bestehende Anfälligkeit von O/W-Emulsionen in Bezug auf eine Zersetzung von Vitamin A und dessen Derivaten hat jedoch noch andere Gründe, wie die vorliegende Erfindung zeigt.
Die Tatsache, daß auch handelsübliche Vitamin A bzw. dessen Derivate enthaltende O/W-Präparate zum Teil eine schlechte Stabilität aufweisen, ist z.B. auf die Verwendung weniger geeigneter Emulgatoren zurückzuführen. Derartige Bestandteile führen dann auch in Anwesenheit der üblichen und geeigneten Stabilisatoren, wie Antioxidantien und Chelatbildnern, zu Produkten mit schlechter Stabilität von Vitamin A und dessen Derivaten.

Wie die Erfindung überraschend zeigt, besteht ein Zusammenhang zwischen der Vitamin A-Zersetzung in O/W-Emulsionen und anderen Emulsionsbestandteilen. Es wurde gefunden, daß der zur O/W-Emulsionsbildung erforderliche Emulgator den Abbau des Vitamin A bzw. dessen Derivaten positiv oder negativ beeinflussen kann.

Die Herstellung chemisch und physikalisch stabiler retinoidhaltiger O/W-Emulsionen gelingt mit geeigneten Emulgatoren sowie gegebenenfalls Antioxidantien und/oder gegebenenfalls Chelatbildnern.

Gegenstand dieser Erfindung sind daher O/W-Emulsionen, die Retinolde (Vitamin A oder Vitamin A-Derivate enthalten sowie spezielle Emulgatoren, gegebenenfalls insbesondere öllösliche Antioxidantien und/oder gegebenenfalls insbesondere wasserlösliche Chelatbildner.

Damit werden erfindungsgemäß die vorstehend genannten Aufgaben gelöst.

Die erfindungsgemäß geeigneten Emulgatoren sind:
a) ein Emulgator auf Basis von Glyceryl Stearat
b) ein Emulgator auf Basis von Glyceryl Stearat und PEG-30 Stearat
c) ein Emulgator auf Basis von Glyceryl Stearat und Ceteth-20
d) ein Emulgator auf Basis von Cetearyl Alcohol und PEG-40 Castor Oil und Sodium Cetearyl Sulfat oder
e) ein Emulgator auf Basis von Sorbitan Stearat.

Die erfindungsgemäßen,speziellen O/W-Emulgatoren können einzeln oder vorzugsweise in Kombinationen oder Mischungen oder Kombinationen von Mischungen eingesetzt werden.

Als Glyceryl Stearat (a) wird vorzugsweise ein Glycerol-mono-di-tri-stearat verwendet, das unter der Handelsbezeichnung 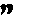 Tegin M" (Fa. Goldschmidt) erhältlich ist. Glyceryl Stearat wird vorzugsweise in Kombination mit einem oder mehreren weiteren Emulgatoren eingesetzt.

Als Glyceryl Stearat und PEG-30 Stearat (b) wird vorzugsweise Polyoxyethylen-5-Glycerinstearat in einer Mischung mit Glyceryl Stearat verwendet, das unter der Handelsbezeichnung 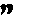 Arlatone 983" (Fa. ICI) erhältlich ist.

Als Glyceryl Stearat und Ceteth-20 (c) wird vorzugsweise Polyoxyethylen-20-Cetylether in einer Mischung mit Glyceryl Stearat verwendet, das unter der Handelsbezeichnung 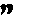 Teginacid H" (Fa. Tego) erhältlich ist.

Als Cetearyl Alcohol und PEG-40 Castor Oil und Sodium Cetearyl Sulfat (d) wird vorzugsweise Cetylstearylalkohol in einer Mischung mit Natriumcetylstearylsulfat und Polyoxyethylen-40-Rizinusöl verwendet, das unter der Handeisbezeichnung 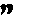 Emulgade F" (Fa. Henkel) erhältlich ist.

Als Sorbitan Stearat (e) wird vorzugsweise Sorbitanmonostearat verwendet, das unter der Handelsbezeichnung 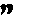 Arlacel 60" (Fa. ICI) erhältlich ist.

Der Anteil eines oder mehrerer Emulgatoren kann 0,05 bis 20 Gewichtsprozent (% m/m) betragen, bezogen auf das Gesamtgewicht der Emulsion.

Vorzugsweise werden mehrere der erfindungsgemäßen Emulgatoren gemeinsam verwendet, und zwar z.B. in Mengen von 0,1 bis 10 % insbesondere von 0,2 bis 5%, jeweils bezogen auf das Gesamtgewicht der Emulsion.

Besonders bevorzugt wird (A) die Mischung von vorzugsweise 0,5 bis 5% Arlatone 983, insbesondere von 1 bis 3% Arlatone 983, mit vorzugsweise 0,5 bis 5% Tegin M, insbesondere von 1 bis 4% Tegin M.

Besonders bevorzugt wird weiterhin (B) die Mischung von vorzugsweise 0,5 bis 4% Teginacid H, insbesondere von 1 bis 3% Teginacid H, mit vorzugsweise 0,5 bis 5% Emulgade F, insbesondere von 1 bis 3% Emulgade F, und vorzugsweise 0,1 bis 4% Arlacel 60, insbesondere von 0,5 bis 3% Arlacel 60.

Besonders bevorzugt wird weiterhin (C) die Mischung von vorzugsweise 0,5 bis 4% Teginacid H, insbesondere von 1 bis 3% Teginacid H, mit vorzugsweise 0,5 bis 5% Emulgade F, insbesondere von 1 bis 3% Emulgade F.

Besonders bevorzugt wird weiterhin (D) die Mischung von vorzugsweise 0,5 bis 5% Emulgade F, insbesondere von 2 bis 4% Emulgade F, und vorzugsweise 0,5 bis 4% Arlacel 60, insbesondere von 1 bis 3% Arlacel 60.

Alle vorstehenden Mengen- und Konzentrationsangaben sind Gewichtsmengen und jeweils bezogen auf das Gesamtgewicht der Emulsion.

Geeignete Retinoide sind z.B. Retinol (Vitamin A₁ bzw. Vitamin A-Alkohol) oder Vitamin A₂, Retinol-Ester, Vitamin A₂-Ester, Vitamin A-Aldehyd (Retinal) oder Vitamin A-Säure (Tretinoin) und deren Ester mit Alkoholen z.B. mit 2-22 Kohlenstoffatomen im Alkoholteil.

Besonders geeignet sind Ester des Vitamins A₂ oder des Retinols mit Carbonsäuren mit vorzugsweise 2-22 Kohlenstoffatomen im Carbonsäureteil, der gesättigt oder ungesättigt sein kann und z.B. 1-5, vorzugsweise eine, zwei oder drei Doppelbindungen besitzen kann.

Besonders bevorzugte Ester sind Retinol (Vitamin A₁)-Acetat und insbesondere Retinol - (Vitamin A₁)-Palmitat.

Mit dem Begriff 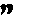 Retinoide" sind alle Isomeren dieser Verbindungen gemeint, insbesondere die all-trans Formen oder die 11-cis- und/oder 13-cis Formen.

Die erfindungsgemäßen Emulsionen können z.B. als Lotionen oder Cremes vorliegen und auch noch Zusatzstoffe oder Hilfsstoffe enthalten.

Die erfindungsgemäßen Emulsionen zeichnen sich durch eine geringe Oxidationsempfindlichkeit aus, so daß Zusätze von Antioxidantien und/oder Chelatbildnern nicht zwingend erforderlich sind. Werden dennoch solche Zusätze gewünscht, können die verwendeten Mengen klein bleiben.

Bevorzugte Antioxidantien sind Butyl-Hydroxy-Toluol, Butyl-Hydroxy-Anisol, Tert.-Butyl-Hydroxy-Chinon, Ascorbinsäure und ihre Derivate wie Ascorbylpalmitat, Mg-Ascorbylphosphat, Tocopherole und ihre Derivate wie alpha-Tocopherylacetat, Gallate wie Propylgallat, Octylgallat, Dodecylgallat oder andere Ester. Antioxidantien können z.B. in Mengen von 0,001 bis 5 Gew.-%, vorzugsweise 0,01 bis 1 Gew.-%, in den Emulsionen enthalten sein.
Weitere geeignete Antioxidantien sind t-Butylhydrochinon, Di-t-Butylhydrochinon und dl-alpha-Tocopherol.

Weiterhin können Chelatbildner, insbesondere in Kombination mit den Antioxydantien, vorzugsweise EDTA, z.B. in Gewichtsmengen von 0, 01 bis 5 Gew.-%, insbesondere 0,1 bis 1 Gew.-%, bezogen auf die gesamte Emulsion enthalten sein. Weitere geeignete Komplexbildner sind Beta-Alanindiessigsäure, Hydroxyethylethylendiamintriessigsäure (Na₃HEDTA), Nitrilotriessigsäure oder Propylendiamintetraessigsäure.

In den erfindungsgemäßen Emulsionen können Fettalkohole und Mineralöle (Paraffine) enthalten sein.

Bevorzugte Fettalkohole sind Myristylalkohol, Cetylalkohol, Stearylalkohol, Cetylstearylalkohol, Behenylalkohol sowie 2-Octyldodecanol (Eutanol G).

Als Paraffin wird vorzugsweise dünnflüssiges Paraffin, gegebenenfalls auch in Kombination mit halbfestem (Vaselin) bzw. festem Paraffin und Mikrowachsen verwendet. Besonders bevorzugt werden geradkettige und verzweigtkettige Paraffine.

Die erfindungsgemäße Emulsion kann zur Einstellung des pH-Wertes von z.B. pH 3 - 8, vorzugsweise pH 4 bis pH 7 Säurezusätze und Zusätze von deren Salzen in geringen Anteilen von beispielsweise etwa 0,01 - 10 Gew.-%, vorzugsweise 0,1 bis 5 Gew.-%, jeweils bezogen auf das Gesamtgewicht der Emulsion, enthalten. Gut geeignet sind Carbonsäuren wie Bernsteinsäure, Milchsäure, Citronensäure oder auch Phosphorsäure und deren Salze als Puffer.

Auch Konservierungsmittel und Stabilisatoren können zugegeben werden. Geeignet sind beispielsweise Stoffe wie Parabene, Benzalkoniumchlorid, p-Chlor-m-kresol, Benzoesäure, Sorbinsäure, Phenoxyethanol, Benzylalkohol, Methyldibromglutarsäurenitril sowie deren Mischungen. Sie können in Mengen von 0,001 - 5 Gew.-%, insbesondere 0,01 bis 2 Gew.-% in der Emulsion enthalten sein.

Die Fettphase der Emulsion besteht beispielsweise aus einem ausgewogenen Gemisch von flüssigen, gerad- oder verzweigtkettigen Paraffinen, natürlichen und synthetischen Estern von verzweigt- oder geradkettigen, gesättigten Fettsäuren mit ein oder mehrwertigen Alkoholen (z.B. Isopopylpalmitat, -myristat, -stearat, -isostearat, Myristylmyristat, Cetylpalmitat, mittelkettige Triglyceride, hydriertes Rizinusöl), femer Fettsäuren (z.B. Linol-, Linolensäuren, Ölsäure), Squalan und/oder Squalen, in Mengen von z.B. jeweils 0,1-30 Gew.-%, vorzugsweise 0,5 bis 10 Gew.-%, insbesondere 1 bis 5 Gew.-%, bezogen auf die gesamte Emulsion. Es können die einzelnen Stoffe oder Gemische verwendet werden.

In den erfindungsgemäßen Emulsionen können weitere Zusatzstoffe oder Hilfsstoffe enthalten sein.

Die Wasserphase enthält beispielsweise mehrwertige Alkohole (z.B. Glycerin, Sorbit, Propylenglycol, 1,3-Butylenglykol, Polyethylenglycole) z.B. in Mengen von jeweils 1-10 Gew.-%.

Alle Mengenangaben, Anteile und Prozentanteile sind auf das Gewicht bezogen, soweit nicht anders angegeben.

Zur Herstellung der Emulsionen werden in an sich bekannter Weise die Bestandteile der Fettphase wie Paraffin und die Emulgatoren geschmolzen und auf erhöhte Temperatur, z.B. 60°C bis 80°C gebracht. Glycerin und Bestandteile der Wasserphase werden bei höherer Temperatur in Wasser gelöst. Die Phasen werden vereinigt und emulgiert. Bei einer niedrigeren Temperatur, z.B. 30°-40°C, wird der Wirkstoff zugegeben. Unter Rühren läßt man die Masse abkühlen.

Aus der erfindungsgemäßen Emulsion, die eine hervorragende Lagerungsstabilität aufweist, wird der Wirkstoff sehr gut absorbiert.

Alle Mengenangaben, Anteile und Prozentanteile sind, soweit nicht anders angegeben , auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

### Chemische Zusammensetzungen der Zusatzstoffe:

- Jonol CP: = 2,6-Di-tert-Butyl-parakresol
- Triton BD: = Ethylendiamintetraessigsäure, Dinatriumsalz
- Eucerit: = destillierte Wollwachsalkohole
- Miglyol 812: = Glyceryl Tricaprylate/Caprate
- Softsan 100: = Triglyceridgemisch v. gesättigten Pflanzenfettsäuren der Kettenlänge C10-C18
- Fitoderm: = gesättigter, verzweigter Kohlenwasserstoff, gewonnen durch Hydrierung von Squalan
- Cetiol SB 45: = 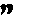 Sheabutter" überwiegend Triglyceride gesättigter Fettsäuren der Kettenlänge C16 - C18
- Lanette O: = Cetylstearylalkohol
- Uniphen P-23: = Mischung aus Methyl-, Ethyl-, n-Propyl-, n-Butyl-, und iso-Butyl-paraben in 2-Phenoxyethanol
- Macadamia Nut Oil: = Macadamianußöl
- Sonnenblumenoel,Raff.: = Sonnenblumenöl
- Natronlauge 45%: = 45%ige NaOH
- Glycerin: = 1,2,3-Propantriol
- Estol 1540: = 2-Ethylhexyl Cocoate
- Carbopol 980: = Carboxylpolymethylen (Polyacrylsäure)

### Beispiel 1

Mit den angegebenen Bestandteilen wird eine O/W-Emulsion hergestellt:

| | Gew.-% |
|---|---|
| Retinyl-Palmitat | 0,5 |
| Arlatone 983 | 2,0 |
| Tegin M | 3,0 |
| Jonol CP | 0,05 |
| Trilon BD | 0,1 |
| Eucerit | 0,1 |
| Miglyol 812 | 1,0 |
| Softsan 100 | 3,0 |
| Fitoderm | 3.0 |
| Cetiol SB 45 | 2,0 |
| Lanette O | 3,0 |
| Uniphen P-23 | 0,65 |
| Macadamia-Nußöl | 5,0 |
| Sonnenblumenoel | 5,0 |
| Natronlauge 45% | 0,2 |
| Glycerin | 5,0 |
| Estol 1540 | 4,0 |
| Carbopol 980 | 0,4 |
| Wasser | ad 100 |

### Beispiel 2

Mit den angegebenen Bestandteilen wird eine O/W-Emulsion hergestellt:

| | Gew.-% |
|---|---|
| Retinyl-Palmitat | 0,5 |
| Teginacid H | 1,3 |
| Arlacel 60 | 1,0 |
| Emulgade F | 2,0 |
| Jonol CP | 0,05 |
| Trilon BD | 0,1 |
| Eucerit | 0,1 |
| Miglyol 812 | 1,0 |
| Softsan 100 | 3,0 |
| Fitoderm | 3.0 |
| Cetiol SB 45 | 2,0 |
| Lanette O | 3,0 |
| Uniphen P-23 | 0,65 |
| Macadamia-Nußöl | 5,0 |
| Sonnenblumenoel, Raff. | 5,0 |
| Natronlauge 45% | 0,2 |
| Glycerin | 5,0 |
| Estol 1540 | 4,0 |
| Carbopol 980 | 0,4 |
| Wasser | ad 100 |

### Beispiel 3

Mit den angegebenen Bestandteilen wird eine O/W-Emulsion hergestellt:

| | Gew.-% |
|---|---|
| Retinyl-Palmitat | 0,5 |
| Teginacid H | 1,3 |
| Emulgade F | 2,0 |
| Jonol CP | 0,05 |
| Trilon BD | 0,1 |
| Eucerit | 0,1 |
| Miglyol 812 | 1,0 |
| Softsan 100 | 3,0 |
| Fitoderm | 3.0 |
| Cetiol SB 45 | 2,0 |
| Lanette O | 3,0 |
| Uniphen P-23 | 0,65 |
| Macadamia-Nußöl | 5,0 |
| Sonnenblumenoel, Raff. | 5,0 |
| Natronlauge 45% | 0,2 |
| Glycerin | 5,0 |
| Estol 1540 | 4,0 |
| Carbopol 980 | 0,4 |
| Wasser | ad 100 |

### Beispiel 4

Mit den angegebenen Bestandteilen wird eine O/W-Emulsion hergestellt:

| | Gew.-% |
|---|---|
| Retinyl-Palmitat | 0,5 |
| Arlacel 60 | 1,5 |
| Emulgade F | 3,0 |
| Jonol CP | 0,05 |
| Triton BD | 0,1 |
| Eucerit | 0,1 |
| Miglyol 812 | 1,0 |
| Softsan 100 | 3,0 |
| Fitoderm | 3.0 |
| Cetiol SB 45 | 2,0 |
| Lanette O | 3,0 |
| Uniphen P-23 | 0,65 |
| Macadamia-Nußöl | 5,0 |
| Sonnenblumenoel, Raff. | 5,0 |
| Natronlauge 45% | 0,2 |
| Glycerin | 5,0 |
| Estol 1540 | 4,0 |
| Carbopol 980 | 0,4 |
| Wasser | ad 100 |

## Patentansprüche

1. O/W Emulsion mit einem Gehalt an Retinolden, dadurch gekennzeichnet, daß sie einen Emulgator oder mehrere Emulgatoren aus der Gruppe, bestehend aus
a) einem Emulgator auf Basis von Glyceryl Stearat;
b) einem Emulgator auf Basis von Glyceryl Stearat und PEG-30 Stearat,
c) einem Emulgator auf Basis von Glyceryl Stearat und Ceteth-20,
d) einem Emulgator auf Basis von Cetearyl Alcohol und PEG-40 Castor Oil und Sodium Cetearyl Sulfat und
e) einem Emulgator auf Basis von Sorbitan Stearat, enthält.

2. Emulsionen gemäß Anspruch 1, dadurch gekennzeichnet, daß sie als Emulgatorengemisch
(A) die Mischung von Arlatone 983 mit Tegin M, oder
(B) die Mischung von Teginacid H mit Emulgade F und Arlacel 60, oder
(C) die Mischung von Teginacid H mit Emulgade F, oder
(D) die Mischung von Emulgade F und Arlacel 60 enthalten.

3. Emulsion gemäß Anspruch 1 oder 2, dadurch gekennzeichnet, daß sie die Emulgatorenmischung (C) enthält.

4. Emulsion gemäß Anspruch 1-3, dadurch gekennzeichnet, daß sie Retinol oder Retinylpalmitat enthält.

5. Emulsion gemäß Anspruch 1-4, dadurch gekennzeichnet, daß sie zusätzlich ein Antioxidans und/oder einen Chelatbildner enthält.
